# EUROPEAN PATENT SPECIFICATION

(11) **EP 4 536 734 B1**
(45) Date of publication and mention of the grant of the patent: **28.01.2026**
(21) Application number: 23728828.7
(22) Date of filing: 08.06.2023
(51) Int. Cl.: C08J 3/20, C07C 235/02, C08J 3/205, C07C 235/10, C08K 5/20, C07C 235/06, C08J 3/075, C08J 3/09, C07C 231/02

(54) **CONTINUOUS OR SEMI-CONTINUOUS PROCESS FOR PRODUCING A PRE-ACTIVATED ORGANOGELATOR PASTE**
KONTINUIERLICHES ODER HALBKONTINUIERLICHES VERFAHREN ZUR HERSTELLUNG EINER VORAKTIVIERTEN ORGANOGELATORPASTE
PROCÉDÉ CONTINU OU SEMI-CONTINU DE PRODUCTION D'UNE PÂTE ORGANOGÉLATRICE PRÉ-ACTIVÉE

(30) Priority: 10.06.2022 EP 22305844
(43) Date of publication of application: 16.04.2025
(73) Proprietor: ARKEMA FRANCE, 92800 Puteaux (FR)
(72) Inventor: AGGARWAL, Sahil, 2600 AA DELFT (NL); VAN ESCH, Johannes Henricus, 2600 AA DELFT (NL); BERNARD, Michael, 60550 VERNEUIL EN HALATTE (FR)
(74) Representative: Arkema Patent
(86) International application number: PCT/EP2023/065420
(87) International publication number: WO 2023/237702

(56) References cited:
- US-A1- 2016 168 079
- US-A1- 2016 312 005
- US-A1- 2018 223 076
- US-A1- 2020 354 545
- US-B2- 9 796 663

## Description

### TECHNICAL FIELD

The present invention pertains to a continuous or semi-continuous process for producing a pre-activated organogelator paste, wherein the process comprises : (1) mixing at least one amide compound with at least one liquid carrier so as to provide a mixture, wherein mixing is carried out at a temperature T1 which is below the activation temperature of the amide compound, (2) continuously flowing said mixture through a heat exchanger to increase its temperature to a temperature T2 that is at least equal to the activation temperature of said mixture, so as to obtain a paste, (3) filling said paste into containers maintained at or above said activation temperature, and (4) retrieving and cooling said containers.

### BACKGROUND OF THE INVENTION

A number of rheology additives have been used to increase the viscosity of mastic, glue or adhesive compositions, coating compositions, such as paints, varnishes, gel coats or inks, or moulding compositions or even electrolytic compositions. Among these, mention can be made of polyamide powders, powders of hydrogenated castor oil-based derivatives, fumed silicas, precipitated calcium carbonates or ground calcium carbonates. Fumed silicas and calcium carbonates are inorganic in nature and require dispersion of the mixture at very high speed. However, these inorganic fillers exhibit problems of stability and sedimentation over time, with resulting negative effects on the mechanical properties of the final system. Another particular drawback of polyamide powders and of powders of hydrogenated castor oil derivatives is the need for activation of the system during the production of the final composition by the user (formulator). This activation requires high-speed shearing, and heating corresponding to temperature rises ranging up to approximately 120°C depending on the products, and also a necessary minimum time dependent on the temperature conditions and system, in order to develop optimum final rheological properties. Examples of documents disclosing fatty acid diamides include US 2016/168079, US 2016/312005; US 2020/354545 and US 9,796,663.

The Applicant has already proposed rheology additives which do not require an activation phase by the end-user, since they are in the form of a pre-activated paste which may simply be blended into the final formulation to provide it with thixotropic properties.

Thus, it has been proposed in EP 1 935 934 a rheology additive comprising at least one fatty acid diamide, introduced in powder form, and at least one organic plasticizer.

An improvement has been proposed, in which the diamide comprises the reaction product of 12-hydroxystearic acid (HSA) and of a linear aliphatic diamine and the plasticizer is replaced with a reactive (meth)acrylic-based solvent comprising at least one cycloaliphatic group (EP 3 371 253).

It has then been suggested to substitute 14-hydroxyeicosanoic acid (14-HEA), which is derived from *Lesquerella* seeds, for 12-HSA, which is derived from castor oil (US 2015/0274644).

All the above additives confer, on the composition into which they are incorporated, a thixotropic behaviour characterized by a marked shear thinning, i.e. a reduction in the viscosity when the shearing increases and then a time-dependent restoration of viscosity (equivalent to a hysteresis effect).

They provide the final composition with excellent properties which are characterized by a high viscosity at rest, good stability of this viscosity on storage, good resistance to sedimentation, ease of application and of extrusion and good resistance to sagging once applied. In addition, these rheology additives have proven to be ready to use in a variety of solvent-based or solvent-free formulations. However, they have always been prepared using a batch process comprising a first step of mixing the diamide with the plasticizer or reactive or non-reactive solvent in an open container at a temperature below room temperature, in order to obtain a homogeneous mixture, followed by a second step of closing said container and heating said mixture in an oven at the activation temperature (i.e. 35-120°C) for 1-100 hours.

This activation step is sometimes difficult to control and reproduce, which may result in a paste which is not always easy to disperse in the final formulation and whose rheological properties may vary depending on the container used to prepare it. In addition, this batch process is expensive in terms of time and energy and the start and stop of production are lengthy, which detrimentally affects its economics. Moreover, only small amounts of diamide and plasticizer or solvent may be prepared, given the fact that the reaction vessel needs to be placed into an oven afterwards. Furthermore, this process restricts the use of solvents to those having a low vapour pressure, which is required to prevent the inner pressure of the container from increasing during heating or decreasing too much during cooling. Because of these safety issues, this process would thus not be applicable to reactive solvents such as methyl methacrylate, unless expensive vessels are used, which are able to withstand such pressures.

It would thus be desirable to provide a cost-effective and safe process for activating such rheology additives which would be more reproducible and versatile and which may be used on an industrial scale, even with solvents having a low vapour pressure.

### SUMMARY

The inventors have shown that it was possible to prepare pre-activated pastes of organogelators based on amide compounds in a reproducible way and on a large scale, using a continuous or semi-continuous process comprising a step of activation performed in continuous flow mode.

This invention is thus directed to a process for producing a pre-activated organogelator paste, wherein the process comprises :
(1) mixing at least one amide compound with at least one liquid carrier so as to provide a mixture, wherein mixing is carried out at a temperature T1 which is below the activation temperature of the amide compound,
(2) continuously flowing said mixture through a heat exchanger to increase its temperature to a temperature T2 that is at least equal to the activation temperature of said amide compound, so as to obtain a paste,
(3) filling said paste into containers maintained at or above said activation temperature, and
(4) retrieving and cooling said containers.

### DETAILED DESCRIPTION

The present invention pertains to a process for producing a pre-activated organogelator paste.

An organogelator (also called rheology additive or a thixotropic agent) may correspond to an organic molecule of low-molecular weight (i.e. less than 2000 g/mol) which is capable of forming a thermoreversible organogel in an organic liquid, in particular at relatively low concentrations (i.e. less than 1% by weight based on the weight of the organic liquid). An organogelator may modify the rheology of a formulation into which it is introduced. In particular, an organogelator may impart a pseudoplastic or thixotropic effect to this formulation. Accordingly, an organogelator may increase the viscosity of the formulation when the formulation is at rest (no shear stress is applied) and lower the viscosity of the formulation when the formulation is subjected to shear stress. The increase and decrease of the viscosity may be determined with respect to a control formulation that does not comprise any organogelator.

In order to exhibit pseudoplastic or thixotropic properties, an organogelator must be activated. This activation generally includes applying specific heating conditions, under shear for a certain period of time. This activation step is generally carried out by the end-user, i.e. by the formulator wishing to impart thixotropic or pseudoplastic properties to a formulation by addition of an organogelator. For example, the end-user may activate the organogelator prior to its introduction in said formulation or the end-user may activate the organogelator *in situ* (directly in said formulation).

Advantageously, the process of the invention provides an organogelator paste in pre-activated form. The wording "pre-activated" should be considered as meaning that the organogelator has thixotropic or pseudoplastic properties and is thus ready for use by the end-user. Accordingly, a pre-activated organogelator may be simply mixed into a formulation and will not require any further activation to exhibit pseudoplastic or thixotropic properties.

The pre-activated organogelator obtained with the process of the invention is in paste form. The wording "paste" should be considered as meaning that the pre-activated organogelator is dispersed in the liquid carried, preferably the pre-activated organogelator is in the form of an organogel. An organogel may be defined as a non-crystalline, non-glassy thermoreversible solid or semi-solid (jelly-like) material composed of an organic liquid entrapped in a three-dimensionally cross-linked network based on self-assembly of a structurant (in the present case an amide compound) by non-covalent interactions (such as hydrogen bonding, van der Waals interactions, π-π stacking ion pairing, solvophobic forces and/or ion coordination). These interactions lead to the formation of a 3D network of microfibrils that immobilize the organic liquid. The organogel may be stable at 25°C for several months, i.e. there is no bulk-phase separation. Upon heating and stirring, the 3D network can be reversibly fragmented, the organogel may become less viscous and when heating under stirring stops, the 3D network of microfibrils may be reformed.

The pre-activated organogelator paste obtained with the process of the invention comprises at least one amide compound and at least one liquid carrier.

The amide compound may be selected from monoamides and/or diamides. A monoamide is a compound bearing one amide bond (-NH-C(=O)-). Such a compound may be the reaction product of at least one monoamine and at last one monocarboxylic acid. A diamide is a compound bearing two amide bonds (-NH-C(=O)-). Such a compound may be the reaction product of at least one diamine and at last one monocarboxylic acid. An asymmetric diamide may be obtained by reaction between a diamine and a mixture of monocarboxylic acids. A mixture of diamides may be obtained when the reaction involves a mixture of diamines and/or a mixture of monocarboxylic acids.

Suitable amide compounds can be obtained by polycondensation between:
a) at least one amine selected from an aliphatic C₂ to C₂₄ monoamine and/or diamine, a cycloaliphatic C₆ to C₁₈ monoamine and/or diamine, an aromatic C₆ to C₁₈ monoamine and/or diamine, and combinations thereof,
b) at least one hydroxylated C₃ to C₃₆ monocarboxylic acid,
c) optionally, at least one saturated linear non-hydroxylated C₂ to C₁₈ monocarboxylic acid.

The amide compound can be optionally combined with hydrogenated castor oil. In this case, the content of the amide compound may range from 10 to 99% by weight, preferably from 20 to 99% by weight, relative to the total weight of the amide compound and the hydrogenated castor oil.

Component (a) comprises at least one amine selected from a monoamine, a diamine and mixtures thereof. As used herein, a monoamine is a compound bearing a single secondary amine group (-NH₂) and a diamine is a compound bearing two secondary amine groups. When component (a) comprises a monoamine, the reaction product comprises a monoamide. When component (a) comprises a diamine, the reaction product comprises a diamide. When component (a) comprises a monoamine and a diamine, the reaction product comprises a monoamide and a diamide. When component (a) comprises a mixture of diamines, the reaction product comprises a mixture of diamides.

Component (a) comprises at least one amine selected from an aliphatic C₂ to C₂₄ monoamine and/or diamine, a cycloaliphatic C₆ to C₁₈ monoamine and/or diamine, an aromatic C₆ to C₁₈ monoamine and/or diamine, and combinations thereof.

As used herein, an aliphatic C₂ to C₂₄ monoamine, respectively diamine, is an acyclic monoamine, respectively diamine, comprising 2 to 24 carbon atoms. The aliphatic monoamine and/or diamine may be linear or branched, preferentially linear. The aliphatic monoamine and/or diamine preferably comprises 2 to 12, more preferably 2 to 8, even more preferentially 2 to 6 carbon atoms.

Examples of aliphatic diamines include 1,2-ethylenediamine, 1,3-propylenediamine, 1,4-tetramethylenediamine, 1,5-pentamethylenediamine, 1,6-hexamethylenediamine, 1,8-octamethylenediamine, 1,12-dodecamethylenediamine and combinations thereof; preferably 1,2-ethylenediamine or 1,6-hexamethylenediamine; more preferably 1,6-hexamethylenediamine.

As examples of linear aliphatic monoamines that are suitable for component (a), mention may be made of: ethylamine, propylamine, butylamine, pentylamine, hexylamine, ethanolamine, and combinations thereof, preferably ethylamine, propylamine, hexylamine or ethanolamine.

As used herein, a cycloaliphatic C₆ to C₁₈ monoamine, respectively diamine, is a monoamine, respectively a diamine, comprising 6 to 18 carbon atoms and at least one non-aromatic ring. The cycloaliphatic monoamine and/or diamine preferably comprises at least one 6-membered non-aromatic ring, which may be bridged or fused with another non-aromatic ring.

As examples of cycloaliphatic diamines that are suitable as component (a), mention may be made of C₆ to C₁₂ cycloaliphatic amines and especially of : cyclohexane-1,3-, -1,4- or -1,2- and in particular -1,3- or -1,4-diamine, 2- or 4-methylcyclohexane-1,3-diamine, isophoronediamine, 1,2-, 1,3- or 1,4-bis(aminomethyl)cyclohexane (derived from the hydrogenation, respectively, of m-, p- or o-xylylenediamine), preferably 1,3- or 1,4-bis(aminomethyl)1,4-cyclohexane, decahydronaphthalenediamine, bis(3-methyl-4-aminocyclohexyl)methane (BMACM), bis(4-aminocyclohexyl)methane (BACM), 1-{[4-(aminomethyl)cyclohexyl]oxy}propan-2-amine, and combinations thereof. The preferred cycloaliphatic diamines are chosen from: cyclohexane-1,3- or -1,4-diamine, 1,2-, 1,3- or 1,4-bis(aminomethyl)cyclohexane, isophoronediamine or bis(4-aminocyclohexyl)methane.

As examples of cycloaliphatic monoamine that are suitable as component (a), mention may be made of cyclohexylamine, isophorylamine, and combinations thereof.

As used herein, an aromatic C₆ to C₁₈ monoamine, respectively diamine, is a monoamine, respectively a diamine, comprising 6 to 18 carbon atoms and at least one aromatic ring. The aromatic monoamine and/or diamine preferably comprises at least one 6-membered aromatic ring, which may be bridged or fused with another aromatic ring. As suitable and preferred examples of aromatic diamines as component (a), mention may be made of C₆ to C₁₄ aromatic amines and especially of: m- or p-xylylenediamine, m- or p-phenylenediamine, m- or p-tolylenediamine, 3,4'- or 4-4'-diaminodiphenylether, 4,4'-diaminodiphenylmethane, and combinations thereof.

As aromatic monoamines, mention may be made of benzylamine, xylylamine and tolylamine.

Preferably, component (a) comprises at least one C₂ to C₂₄, in particular C₂ to C₁₂, more particularly C₂ to C₈ linear aliphatic diamine; and optionally at least one other diamine selected from a cycloaliphatic C₆ to C₁₈ diamine, an aromatic C₆ to C₁₈ diamine, and combinations thereof.

Component (b) comprises at least one hydroxylated C₃ to C₃₆ monocarboxylic acid, i.e. a compound comprising 3 to 36 carbon atoms, a single carboxy group (-COOH) and one or more hydroxy groups (-OH), preferably a single hydroxy group. This hydroxylated C₃ to C₃₆ monocarboxylic acid may be saturated or unsaturated (in this case preferably in *trans* form), preferably saturated.

The hydroxylated monocarboxylic acid is preferably selected from C₁₆ to C₂₂ monohydroxylated monocarboxylic acids, preferably 14-hydroxyeicosanoic acid and/or 9-, 10- and/or 12-hydroxystearic acid (9-HSA, 10-HSA and/or 12-HSA). 12-hydroxystearic acid may be obtained by hydrogenation of castor oil followed by hydrolysis of the resulting hydrogenated castor oil. 14-hydroxyeicosanoic acid may be derived from lesquerolic oil produced by extraction from *Lesquerella* seeds and thus from the cultivation of *Lesquerella*. *Lesquerella* oil is typically subjected to transesterification with methanol, then the resulting product is hydrogenated and finally hydrolyzed to give 14-hydroxyeicosanoic acid. An example of polyhydroxylated monocarboxylic acid is 9,10-dihydroxystearic acid.

Preferably, component (b) comprises 12-hydroxystearic acid.

Component (c), which is optional, comprises at least one saturated linear non-hydroxylated C₂ to C₁₈ monocarboxylic acid, i.e. a linear saturated compound comprising 2 to 18 carbon atoms, a single carboxy group (-COOH) and no hydroxy groups (-OH). The non-hydroxylated monocarboxylic acid preferably comprises 2 to 15 carbon atoms, more preferably 6 to 12 carbon atoms. As examples of non-hydroxylated monocarboxylic acids suitable for component (c), mention may be made of: acetic, propanoic, butanoic, pentanoic, hexanoic, heptanoic, octanoic, nonanoic, decanoic, undecanoic, dodecanoic, and stearic acid. The following non-hydroxylated monocarboxylic acids are preferred: hexanoic, octanoic, nonanoic and decanoic acids.

According to an embodiment of this invention, the amide compound is a diamide compound obtained by polycondensation between 12-hydroxystearic acid and 1,6-hexamethylenediamine.

The polycondensation reaction between the above components (a) and (b) and optionally (c), may be performed at a temperature ranging from 140 to 250°C, preferably from 150 to 200°C. The reaction is preferably conducted under inert atmosphere. The molar ratio between the amine groups of component (a) and the carboxy groups of components (b) and (c) typically ranges from 0.9 to 1.1 and is preferably of 1:1. In addition, if component (c) is present, the molar ratio of component (b) to component (c) generally ranges from 1:2 to 4:1.

This polycondensation reaction allows the recovery of an amide compound. By this expression, it is intended to designate one or more monoamide compounds, one or more diamide compounds and mixtures thereof. Preferred amide compounds are diamide compounds optionally mixed with monoamide compounds.

The amide compound obtained after the polycondensation reaction is typically in solid form. In order to facilitate the mixing of the amide compound with the liquid carrier in the first step of the process of the invention, the amide compound is preferably in particulate form. In particular, the amide compound may be in powder form or in micronized form. For example, the amide compound may be micronized by mechanical grinding (ball milling) or by air jet. Preferably the amide compound has a volume particle size Dv(90) of less than 30 µm, preferentially less than 25 µm, more preferentially less than 20 µm, even more preferentially of less than 15 µm. In particular, the Dv(90) may be from more than 5 µm to less than 30 µm. The Dv(90) particle size may be understood as the volume-based size distribution that includes 90% of the particles present in a given sample. Said size can be determined by laser diffraction using the method described herein.

This amide compound may then be transformed into a pre-activated organogelator paste using the process according to the invention. In particular, the amide compound may be mixed with a liquid carrier and heated to a temperature equal to or greater than its activation temperature to allow self-assembly of its molecules by non-covalent bonds in order to obtain microfibrils.

The first step of the process of the invention involves the use of a liquid carrier. The liquid carrier may be used to at least partly solubilize or disperse the amide compound.

The liquid carrier may be any organic compound which is liquid at temperature T1 (for example 30°C). Preferably, the liquid carrier is an organic compound which is liquid throughout the temperature range of 0°C to T1 (for instance 0 to 30°C). Preferably, the liquid carrier is an organic compound which is liquid throughout the temperature range of T1 to T2 (for instance 30°C to 120°C).

The liquid carrier may comprise one or more plasticizers, one or more reactive solvents and one or more non-reactive solvents or mixtures thereof. In this description, the wording "a plasticizer" or "a solvent" thus covers both a single compound and a mixture of compounds, unless otherwise specified.

As used herein, the term "solvent" means a compound that is able to at least partly solubilize the amide compound, optionally under heating conditions. The solvent is preferably an aprotic organic solvent. Even more preferably, the solvent is a polar aprotic organic solvent.

As used herein, the term "reactive solvent" means a solvent that comprises a reactive functional group, i.e. a functional group that is able to react with at least one component of the formulation into which the pre-activated organogelator paste is added. For example, a reactive solvent may comprise at least one polymerizable carbon-carbon double bond and/or at least one epoxide ring.

As used herein, the term "non-reactive solvent" means a solvent which is inert towards the components of the formulation into which the pre-activated organogelator paste is added. Typically a non-reactive solvent is devoid of reactive functional groups as defined above. As used herein, the term "plasticizer" means a compound that is able to modify the mechanical and/or thermal properties of the formulation into which the pre-activated organogelator paste is added, optionally under heating conditions. For example, a plasticizer may have one or more of the following effects on the formulation into which the pre-activated organogelator paste is added: decrease of the viscosity, decrease of the glass transition temperature, increase of the flexibility, etc....

Examples of non-reactive solvents are: xylene; alcohols such as methanol, ethanol, butanol and benzyl alcohol ; cyclic saturated hydrocarbons such as cyclopentane, cyclohexane, methylcyclohexane, ethylcyclohexane, dimethylcyclohexane, trimethylcyclohexane and decalin ; alkylesters of monocarboxylic acids such as methyl formate, methyl acetate, ethyl acetate, butyl acetate, hexyl acetate, heptyl acetate, methyl propionate, ethyl propionate, amyl propionate and ethyl ethoxypropionate ; alkylesters of dicarboxylic acids, such as methyl glutarate, methyl succinate and methyl adipate ; lactones, such as γ-butyrolactone; ethers, such as dimethoxyethane (DME), methyl ethers of oligoethylene glycols of 2 to 5 oxyethylene units, 1,3-dioxolane, dioxane, dibutyl ether and tetrahydrofuran; ketones such as cyclohexanone; phosphoric acid esters or sulfite esters ; nitriles, such as acetonitrile, pyruvonitrile, propionitrile, methoxypropionitrile, dimethylaminopropionitrile, butyronitrile, isobutyronitrile, valeronitrile, pivalonitrile, isovaleronitrile, glutaronitrile, methoxyglutaronitrile, 2-methylglutaronitrile, 3-methylglutaronitrile, adiponitrile and malononitrile; carbonates such as ethylene carbonate, propylene carbonate, butylene carbonate, dimethyl carbonate, diethyl carbonate, ethyl methyl carbonate, diphenyl carbonate, methyl phenyl carbonate, dipropyl carbonate, methyl propyl carbonate, ethyl propyl carbonate, vinylene carbonate, fluoroethylene carbonate, trifluoropropylene carbonate; and mixtures thereof.

Examples of mixtures of non-reactive solvents are mixtures of xylene with either ethanol or butanol, for instance in a 1:1 to 4 :1 weight ratio.

In case a reactive solvent is used, it is typically chosen from an ethylenically unsaturated compound (i.e. a compound comprising a polymerizable carbon-carbon double bond) or an epoxide (i.e. a compound comprising an epoxy ring). A polymerizable carbon-carbon double bond is a carbon-carbon double bond that can react with another carbon-carbon double bond in a polymerization reaction. A polymerizable carbon-carbon double bond is generally comprised in a group selected from acrylate (including cyanoacrylate), methacrylate, acrylamide, methacrylamide, maleate, fumarate, itaconate, allyl, propenyl, vinyl and combinations thereof, preferably selected from acrylate, methacrylate, and vinyl, more preferably selected from acrylate and methacrylate.

In particular, the reactive solvent may comprise one or more ethylenically unsaturated compounds selected from a (meth)acrylic monomer, a styrenic monomer, a vinylic monomer, an olefinic monomer, an unsaturated polyacid or a derivative thereof, and mixtures thereof.

As used herein the term "(meth)acrylic monomer" means a monomer that comprises a carbon-carbon double bond conjugated with a carbonyl (-C(=O)-) bond. Such monomers typically comprise a (meth)acryloyl group. Preferably, the (meth)acrylic monomer is a monofunctional (meth)acrylic monomer, i.e. it comprises only one (meth)acryloyl group. The term (meth)acryloyl group is indifferently used to refer to an acryloyl group (-C(=O)-CH=CH₂) or a methacryloyl group (-C(=O)-C(CH₃)=CH₂). The (meth)acrylic monomer may be selected from alkyl (meth)acrylates (in particular methyl (meth)acrylate, ethyl (meth)acrylate, butyl (meth)acrylate, tert-butyl (meth)acrylate or 2-ethylhexyl (meth)acrylate); cycloaliphatic (meth)acrylates (in particular dicyclopentadienyl (meth)acrylate, norbornyl (meth)acrylate, isobornyl (meth)acrylate (IBO(M)A), tert-butyl cyclohexanol (meth)acrylate (TBCH(M)A), tricyclodecane methanol mono(meth)acrylate and 3,3,5-trimethylcyclohexanol (meth)acrylate (TMCH(M)A)); hydroxyalkyl (meth)acrylates (in particular 2-hydroxyethyl (meth)acrylate); (meth)acrylic acid; (meth)acrylamide; (meth)acrylonitrile; or mixtures thereof.

As used herein, the term "styrenic monomer" refers to a monomer that contains a carbon-carbon double bond in alpha position to an aromatic ring. The styrenic monomer may be selected from styrene, alpha-methylstyrene, tert-butylstyrene, ortho-, meta- or para-methylstyrene, ortho-, meta- or para-ethylstyrene, o-methyl-p-isopropylstyrene, p-chlorostyrene, p-bromostyrene, o,p-dichlorostyrene, o,p-dibromostyrene, ortho-, meta- or para-methoxystyrene, optionally substituted indenes, optionally substituted vinyl naphthalenes, acenaphthylene, diphenylethylene, vinyl anthracene or mixtures thereof.

As used herein, the term "vinylic monomer" refers to a monomer that contains a vinyl group (-CH=CH₂) that is not conjugated with a carbonyl (C=O) group or an aromatic group. Vinylic monomers are distinct from olefinic monomers in that they comprise at least one heteroatom (i.e. an atom other than carbon or hydrogen). The vinylic monomer may be selected from vinyl halides (in particular vinyl chloride); vinyl esters (in particular vinyl acetate, vinyl formate, vinyl propionate, vinyl butyrate, vinyl isobutyrate, vinyl pivalate, vinyl pentanoate, vinyl hexanoate, vinyl octanoate, vinyl 2-ethylhexanoate, vinyl pelargonate, vinyl laurate, vinyl stearate, and vinyl versatate, i.e. esters of branched monocarboxylic acids having 6, 9, 10 or 11 carbon atoms, available under references VeoVa^{®} EH, VeoVa^{®} 9, VeoVa^{®} 10 or VeoVa^{®} 11 from Hexion); vinyl ethers (in particular methyl-, ethyl-, butyl- or iso-butyl vinyl ether) or mixtures thereof.

As used herein, the term "olefinic monomer" refers to a non-aromatic hydrocarbon monomer that contains one or more carbon-carbon double bonds. The olefinic monomer may be selected from ethylene, propene, 1-butene, isobutylene, diisobutylene, 1-nonene, 1-decene, butadiene, or mixtures thereof.

As used herein, the term "unsaturated polyacid" means a compound comprising at least one carbon-carbon double bond and at least two carboxy groups, the carbon-carbon double bond being conjugated with at least one of the carboxy groups. The term "unsaturated polyacid derivative" means a compound capable of yielding an unsaturated polyacid in situ, for example by hydrolysis or ring opening. Examples of suitable unsaturated polyacid derivatives include alkyl esters of unsaturated polyacids and cyclic anhydrides. The unsaturated polyacid or derivative thereof may be selected from fumaric acid, maleic acid, itaconic acid, aconitic acid, mesaconic acid, anhydrides thereof and mixtures thereof.

Alternatively, the reactive diluent may be a cardanol derivative or a glycidyl ether.

As used herein, the term "cardanol derivative" means a compound obtained by chemical modification of cardanol. Cardanol is a biobased phenolic lipid extracted from the cashew nut shell liquid (a by-product of cashew nut processing). Cardanol comprises a phenol ring substituted with an unsaturated fatty chain and may be chemically modified by a variety of reactions. The OH group of the phenolic ring may be transformed into an ester group, a phosphate group, an ether or a glycidyl ether. Alternatively, the unsaturated fatty chain may be epoxidized. Examples of suitable cardanol derivatives include-{3-[(8E)-pentadec-8-en-1-yl]phenoxymethyl}oxirane (Cardolite NC-513) or [(7Z)-pentadec-7-en-1-yl]phenol (Cardolite NX-2022).

As used herein, the term "glycidyl ether" designates a compound comprising a glycidyl ether group of formula

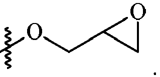

Glycidyl ethers may be obtained by reacting an alcohol or a polyol with epichlorhydrin. Examples of suitable glycidyl ethers include C4-C20 alkyl glycidyl ethers (such as octyl, decyl, dodecyl, tetradecyl or hexadecyl glycidyl ether), 1,2- or 1,3-propylene glycol diglycidyl ether, 1,2-, 1,3- or 1,4-butanediol diglycidyl ether, 1,5-pentanediol diglycidyl ether, 1,6-hexanediol diglycidyl ether, 1,8-octanediol diglycidyl ether, 1,9-nonanediol diglycidyl ether, 1,10-decanediol diglycidyl ether, 1,12-dodecanediol diglycidyl ether, 2-methyl-1,3-propanediol diglycidyl ether, neopentyl glycol diglycidyl ether, 2,2-diethyl-1,3-propane diol diglycidyl ether, 3-methyl-1,5-pentanediol diglycidyl ether, 3,3-dimethyl-1,5-pentanediol diglycidyl ether, 2,4-diethyl-1,5-pentanediol diglycidyl ether, 3,3-butylethyl-1,5-pentane diol diglycidyl ether, glycerol triglycidyl ether, trimethylolmethane triglycidyl ether, trimethylolethane triglycidyl ether, trimethylolpropane triglycidyl ether, di(trimethylolpropane) tetraglycidyl ether, pentaerythritol tetraglycidyl ether, cyclohexane diglycidyl ether, cyclohexane-1,4-dimethanol diglycidyl ether, tricyclodecane dimethanol diglycidyl ether, isosorbide diglycidyl ether, pyrocatechol diglycidyl ether, resorcinol diglycidyl ether, cardol diglycidyl ether, phloroglucinol triglycidyl ether, pyrogallol triglycidyl ether, tris(hydroxyphenyl)methane triglycidyl ether, tris(hydroxyphenyl)ethane triglycidyl ether, a hydrogenated bisphenol diglycidyl ether, a bisphenol diglycidyl ether, glycidyloxypropyl trimethoxysilane, and mixtures thereof.

According to an embodiment, the reactive solvent is a monofunctional (meth)acrylic monomer, preferably an alkyl (meth)acrylate or a cycloaliphatic (meth)acrylate. The pre-activated organogelator paste obtained from these monomers is suitable, in particular, as an additive for compositions based on an epoxyamine two-component reactive system, in which the reactive solvent is able to react with the amine (curing agent) of the epoxyamine system.

In the case where a plasticizer is used, said plasticizer is preferably selected from a polar organic plasticizer which generally comprise at least one polar group selected from an ether group and/or an ester group and/or an epoxy group.

Plasticizers comprising at least one ether group may be chosen from polyethers, such as homopolymers and/or copolymers of ethylene oxide and/or of propylene oxide and/or a blend of said polyethers (homopolymers and/or copolymers) and/or derivatives thereof, these derivatives comprising, *inter alia,* said polyethers blocked at the chain end with a C₁ (methoxy) to C₄ (butoxy) alkoxy group, or with a C₂ (acetate) to C₄ (butyrate) ester group, said polyethers having a weight-average molecular weight Mw ranging from 150 to 6000 and preferably from 1000 to 3000. The wording "copolymer" is to be interpreted as comprising both random copolymers and block copolymers.

Plasticizers comprising at least one ester group may be chosen from monoesters and/or polyesters (polyfunctional esters) obtained from C₄ to C₂₁ alcohols, which alcohols are optionally alkoxylated, for example with 1 to 10 alkoxy units chosen from oxyethylene (OE) and/or oxypropylene (OP) units, and from mono- or polyacids with a functionality ranging from 1 to 4, selected from: (a) organic acids chosen from aromatic acids having a chain length (without -CO₂H) ranging from C₆ to C₁₀ and/or aliphatic acids having a chain length (without -CO₂H function) ranging from C₄ to C₁₈ and/or cycloaliphatic acids having a chain length (without -CO₂H function) ranging from C₆ to C₁₀, and (b) inorganic acids.

The esters of aromatic acids can be chosen from phthalic esters (phthalates) and trimellitic esters (trimellitates). The esters of aliphatic acids can be chosen from adipic esters (adipates), citric esters (citrates), sebacic esters (sebacates) and azelaic esters (azelates). The esters of cycloaliphatic acids can be chosen from tetrahydrophthalic esters (tetrahydrophthalates) and hexahydrophthalic esters (hexahydrophthalates). The esters of inorganic acids can be chosen from sulphonic esters (sulphonates), in particular C₁₀ to C₂₁ alkyl sulphonates, sulphuric esters (sulphates), sulphinic esters (sulphinates), phosphoric esters (phosphates), phosphonic esters (phosphonates) and phosphinic esters (phosphinates).

Finally, in case the plasticizer comprises at least one epoxy group, it may be chosen from epoxidized vegetable oils such as epoxidized soybean oil or epoxidized linseed oil, and epoxidized fatty acid alkyl esters such as epoxidized methyl oleate, epoxidized iso-amyl stearate or epoxidized 2-ethylhexyl stearate.

Among preferred plasticizers, mention may be made of those comprising at least one C₆ to C₁₀ aromatic acid ester group, in particular plasticizers selected from mono- and/or dialkyl phthalates, and even more preferentially from dialkyl phthalates or hexahydrophthalates, wherein said alkyls are identical or different and chosen from C₇ to C₁₈ alkyls, and preferably C₁₀ to C₁₂ alkyls. The most preferred plasticizers in this family of (dialkyl (hexahydro)phthalates) are diisoundecyl phthalate and diisononyl hexahydrophthalate. The most preferred polyethers are the polyethers which are homopolymers of propylene oxide (polypropylene glycols) with a weight-average molecular weight Mw ranging from 1000 to 3000, and more particularly polypropylene glycol (PPG) with an Mw equal to 2000, and/or derivatives thereof chosen from monoesters, preferably C₂ to C₄ monoesters, or C₁ to C₄ monoethers, such as monomethoxylated or monoethoxylated polypropylene glycol.

Usually, the amide compound is present in a weight ratio, relative to the liquid carrier, ranging from 5:1 to 1:20, preferably from 2:1 to 1:15, more preferably from 1:1 to 1:10 and even more preferably from 1:3 to 1:6.

More specifically, the process of this invention comprises a first step of mixing the amide compound with the liquid carrier to provide a mixture (also referred to as the mixing step). This step may be carried out in a tank or reactor equipped with an agitation system, such as a rotating impeller and optionally one or more baffles. For example, the amide compound and the liquid carrier may be stirred at 1000-3000 rpm, preferably 1500-2000 rpm. Stirring may be carried out for 10 minutes to 1 h, for instance 15 to 30 minutes. Alternatively, the mixing step may be carried out in a static mixer, for example in a tube or pipe equipped with stationary elements that redistribute the fluid in the directions transverse to the main flow (i.e. in the radial and tangential directions). For example, the amide compound may be introduced in a continuous stream of liquid carrier flowing through a static mixer. The mixing step is carried out at a temperature T1 that is lower than the activation temperature of the amide compound. Preferably, temperature T1 should be at least 10°C lower, or at least 20°C lower, or at least 30°C lower, than the activation temperature of the amide compound. Such temperatures are advantageously selected so that the viscosity of the mixture does not substantially increase during the mixing step (no premature activation of the amide compound). In one embodiment, T1 is at most 30°C, preferably at most 25°C, even more preferably from 0 to 25°C. The temperature of the mixture during the mixing step may be regulated to remain below T1 by any suitable means, for example a cold bath or a double-jacket cooling system. Said temperature regulation may be useful to avoid overheating created by shear during the mixing of the amide compound and/or to avoid premature and uncontrolled activation of the amide compound. The mixing step may be carried out until a homogeneous mixture is obtained. The homogeneity of the mixture may be characterized by an absence of sedimentation or an absence of visible particles when said amide compound is mixed with the liquid carrier at a shear rate preferably of between 2 and 6 m.s⁻¹, for a preparation on the laboratory scale with regard to a volume not exceeding 1 litre.

In the second step of the process according to this invention, the above mixture is continuously flowed through a heat exchanger to increase its temperature to a temperature T2 (also referred to as the activation step). Accordingly, the temperature of the mixture as it enters the heat exchanger may be at most T1, which is below the activation temperature of the amide compound (for example 0 to 30°C), and the temperature of the mixture as it exits the heat exchanger is T2 (for example 35 to 120°C). Temperature T2 is at least equal to the activation temperature of the amide compound, i.e. the temperature sufficient for forming an organogel. Said activation temperature may be easily determined by the skilled artisan depending on the nature of the amide compound and of the liquid carrier used. For example, the skilled artisan may apply heat to a mixture of the amide and the liquid carrier in a container and see when the gel point is reached. Temperature T2 is typically within the range of 35 to 120°C, for instance 50 to 100°C. During its stay in the heat exchanger, the temperature of the mixture is progressively increased by means of a heat transfer fluid circulating in the heat exchanger. The temperature of the heat transfer fluid as it enters the heat exchanger may be equal to temperature T2. The heat transfer fluid may circulate in co-current flow or counter-current flow with respect to the flow of the mixture. When the mixture exits the heat exchanger, it is in the form of a paste. The residence time of the mixture in the heat exchanger depends, among others, on the flow rate of the mixture, the targeted activation temperature, the flow of the heat transfer fluid, and the dimensions of the heat exchanger. Typically, the residence time may be less than 1 hour, preferably from 30 seconds to 50 minutes, still preferably from 1 minute to 30 minutes. This activation step may be carried out in any suitable heat exchanger allowing a continuous flow of the mixture and allowing to regulate the residence time of the mixture. For instance, the heat exchanger may be selected from a double pipe heat exchanger, a coil heat exchanger, a shell-and-tube heat exchanger or a plate heat exchanger. The flow rate of the mixture may be adjusted to a set value, depending on the dimensions of the heat exchanger, such that the residence time is within the optimal range that has been predetermined.

The continuous flowing of the mixture through the heat exchanger may be driven by a pump. The pump may be placed upstream of the heat exchanger. The type of pump used is not critical so that, for example, non-chokable pumps, impeller-type pumps, disc-flow pumps, rotating piston pumps, eccentric single-rotor screw pumps, cylindrical diaphragm pumps, etc. can be used. It is also of no critical importance whether the mixture is pumped in laminar or turbulent flow.

The paste material thus obtained after the second step is filled into containers maintained at a temperature equal to or greater than the activation temperature, such as temperature T2. The containers are then retrieved and cooled down before use in a formulation. The process of this invention thus allows preparing a pre-activated organogelator paste with a continuous process. In an embodiment of this invention, a semi-continuous process may be used, in case the paste is not fully activated when it exits the heat exchanger. This process may thus further comprises a step of transferring the containers to an oven for completing the activation of the amide compound ; typically at the temperature set previously (temperature T2) and for a predetermined period of time, before they are retrieved and cooled down.

In any case, it is considered that activation is complete when the gel point is reached, in other words when the consistency of the paste of reaches a plateau value without significantly changing thereafter. The gel thus activated (also called pre-activated organogelator paste) can be characterized firstly by means of a quite simple test using a wooden spatula introduced into the gel or the paste to be tested and which remains vertical without moving, if the tested product is sufficiently activated with said gel or paste remaining single-phase, and does not exude the liquid carrier. More specifically, the viscosity of the activated paste is such that the maximum penetration value of the paste formed is less than 15 mm, preferably less than 10 mm, measured according to ASTM standard D 217 (2010) with the following conditions: temperature of 23°C, cone of 30 degrees, test speed of 5 mm/s with penetration force of 0.4 N. Alternatively, it can be considered that the suitable viscosity has been reached when the dynamic elastic modulus E' of the paste is of at least 10⁴ Pa at 23°C and 1 Hz.

An example of a setup that may be used to perform the process of this invention is shown on the attached Figure.

As shown on this Figure, this equipment comprises a reaction vessel 1 provided with stirring means 2, such as a stainless steel or copper tank with a rotating impeller. At the laboratory scale, a roundbottom vessel equipped with a PTFE stirrer may be used. This reaction vessel is in fluid connection with containers (not represented) respectively comprising the amide compound and liquid carrier and fed to the reaction vessel 1 via pumps (not represented). The reaction vessel is immersed in a cold bath so as to maintain the temperature inside the reaction vessel at a temperature T1 ranging from 0 to 5°C. The homogeneous mixture prepared in the reaction vessel 1 is transferred via a pump 3, for instance a peristaltic pump, to a heat exchanger 4, such as a coil heat exchanger, connected to a hot bath 5 maintained at 60-65°C. The heat exchanger is mounted such that the mixture flows through the coils and the hot water flows around the coils in a closed loop. The mixture is maintained in continuous flow within the heat exchanger until it reaches a temperature T2 that is at least equal to the activation temperature, in the present case 60-65°C. The residence time of the mixture within the heat exchanger 4 is controlled by adjusting the flow rate of the mixture by means of the pump 3. An activated paste is thus produced, which exits the heat exchanger 4 via a duct 6 and is split into containers 8, such as sample vials, three of which are represented on this Figure. These containers are maintained at temperature T2, i.e. 60-65°C, by means of a hot bath 7, and they are collected once they are filled. An oven 9 is optionally provided to maintain the containers at temperature T2 in order to complete the activation if needed.

The pre-activated organogelator paste as described above may be added to a formulation which may be a coating composition, a moulding composition or an electrolyte composition. The coating composition may be applied onto various substrates and used as protective coating and/or decorative coating and/or surface treatment coating. Said coating composition may be selected from paints, varnishes, pigmented or unpigmented gel coats, inks, plastisols based on PVC. Alternatively, it may be a mastic, glue, adhesive or sealant composition. In another embodiment, the coating composition may be a cosmetic composition such as a nail lacquer. In still another embodiment, the coating composition may be a photocurable composition for stereolithography or for 3D printing of objects, in particular by inkjet. This coating composition may be applied to the substrate by spraying, for instance with a spray gun, or with a brush or a roller. In case the formulation is a moulding composition, it may be selected from moulding compositions for composites, for example of SMC or BMC or laminated type, such as boat hulls or composite panels. Examples of electrolyte compositions for lithium-ion batteries have been described in FR 3 103 637. They may comprise a lithium salt and electrolytic additives such as fluoroethylene carbonate (FEC) or vinylene carbonate.

The pre-activated organogelator paste can be present in these formulations at a content by weight ranging from 1 to 30%, and preferably from 2 to 25%, and said amide compound can be present as dry active matter at a content by weight ranging from 0.2 to 8%, and preferably from 1 to 6%, relative to the weight of said formulation.

The formulation can comprise other components, such as, for example, fillers, plasticizers, wetting agents or else pigments.

Usually, the formulation includes an organic reactive binder. The pre-activated organogelator paste described above may be used both with organic reactive binders which are crosslinked by the radical route, whether under radiation or thermally or by an initiation system based on peroxide or hydroperoxide, and with organic reactive binders which are crosslinked by reaction with reactive amino components by Michael addition reaction, for instance an epoxyamine two-component reactive binder.

Thus, according to an embodiment, the organic reactive binder is crosslinkable, either thermally or by irradiation under radiation such as UV radiation (in the presence of at least one photoinitiator) and/or EB radiation (electron beam, without initiator), including self-crosslinkable at ambient temperature, or is non-crosslinkable. The organic reactive binder may be single-component crosslinkable (just one reactive component) or two-component crosslinkable (based on two components which react with one another by mixing during use).

Said organic reactive binder can be selected from at least one epoxy/amine (crosslinkable two-component) reactive system, an unsaturated polyester, a vinyl ester, an epoxidized resin, a reactive silicone resin, an alkyd grafted with a polyester or a polyamide or of diurea/diurethane type, or an ungrafted alkyd, a polyurethane or a silicone, a crosslinkable two-component polyurethane, a polysiloxane, a polysulphide polymer, a reactive acrylic polymer, a (meth)acrylate polyfunctional oligomer or acrylic oligomer or allylic polyfunctional oligomer, a butyl rubber, polychloropene or SBR type elastomer, or a silylated prepolymer, preferably a silylated polyether or a silylated polyurethane, or a silylated polyether/urethane with an -OH or -CO₂H function.

In a more particular case, said organic reactive binder can be selected from the following crosslinkable two-component reactive systems: epoxy/amine or epoxy/polyamide systems comprising at least one epoxy resin comprising at least two epoxy groups and at least one amino or polyamide compound comprising at least two amine groups, polyurethane systems comprising at least one polyisocyanate and at least one polyol, polyol/melamine systems, and polyester systems based on at least one epoxy or on one polyol which reacts with at least one corresponding acid or anhydride.

According to other specific cases, said organic reactive binder may be a crosslinkable two-component polyurethane system or a crosslinkable two-component polyester system starting from an epoxy/carboxylic acid or anhydride reaction system, or from a polyol/carboxylic acid or anhydride system, or a polyol/melamine reaction system in which the polyol is a hydroxylated acrylic resin, or a polyester or a polyether polyol.

According to an embodiment, the formulation is a coating composition selected from a mastic, glue, adhesive or sealant composition which is self-crosslinkable and based on polyether/silane or on polyurethane/silane organic reactive binders.

Typically, the formulation is prepared by adding the pre-activated organogelator paste described above to the remaining constituents of said formulation, then homogenizing the mixture with a kneader and/or planetary mixer and/or high-speed disperser, without having any need, in this step, to apply a heat activation treatment, wherein "high speed" means tangential speeds ranging from 2 to 15 m.s⁻¹.

### BRIEF DESCRIPTION OF THE DRAWINGS

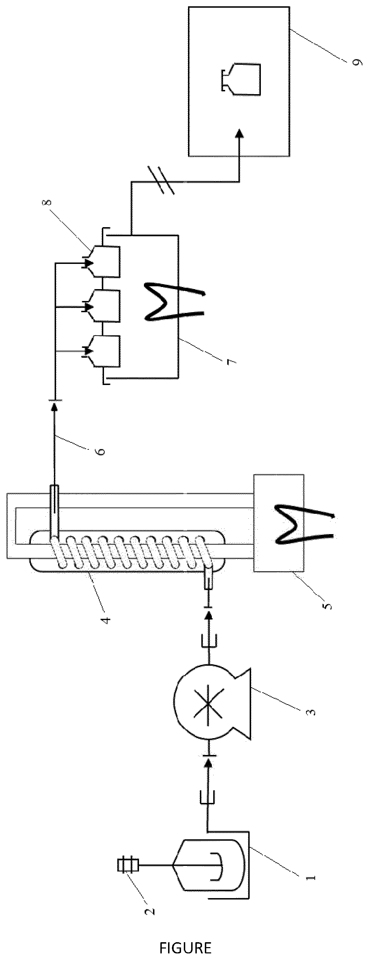

Figure 1 represents an installation which may be used for conducting the continuous or semi-continuous process of this invention.

### EXAMPLES

This invention will be better understood in light of the following examples which are given for illustrative purposes only and do not intend to limit the scope of the invention, which is defined by the attached claims.

### Example 1 :

### Products:

| Raw Materials | Description | Supplier |
|---|---|---|
| 12-HSA | 12-hydroxystearic acid technical grade (could contain <10 wt% stearic acid, <5 wt% estolides). | Jayant Agro |
| HMDA | 1,6-hexamethylenediamine | Aldrich |
| Amide compound | 12HSA-HMDA-12HSA synthesized and micronized according to Patent WO 2017/077220. | Arkema |
| Xylene | Solvent | Aldrich |
| Ethanol | Solvent | Aldrich |

### Synthesis example 1: Preparation of diamide compound:

49.96 grams of HMDA (i.e. 0.43 mol, 0.86 amine equivalent) and 271.1 grams of 12-HSA (i.e. 0.86 mol, 0.86 acid equivalent) were placed under a nitrogen atmosphere in a 1 liter roundbottomed flask equipped with a thermometer, Dean-Stark apparatus, a condenser and a stirrer. The mixture was heated to 200°C under a stream of nitrogen. The water was collected in the Dean-Stark apparatus at 150°C. The reaction was monitored via the acid number and the amine number. When the acid and amine number values were less than 10 mg KOH/g, the reaction mixture was cooled to 150° C and then discharged into a silicone-treated mold. Once cooled to room temperature, the product was micronized mechanically by milling and screening to obtain a fine and controlled particle size with a volume particle size Dv90 below 10 µm. The particle size may be measured by dry laser granulometry using a Mastersizer MS3000 (Malvern Panalytical) apparatus with the following conditions:
Range of measure : 0.1 to 3500 µm
Optical model : 1.30-0i
Analysis mode : MS3000 : standard / peak of fine powder
Obscuration : 0,3 to 10 %
Number of measures : 3
Lab Equipment:

### - Comparative example

The following equipment was used to reproduce the process disclosed in [0122]-[0123] of US2015/0274644 :

| Equipment | Description | Parameter | Supplier (ref) |
|---|---|---|---|
| Oven | Ventilated Oven - Universal | 60°C | Memmert |
| Speed mixer | ATP Dispermill | 1500 rpm | ATP Engineering B.V. (Dispermill 2075 Yellow-Line HS) |
| Dispersing disk | Paddle | Diameter: 30mm | ATP Engineering B.V. (30-1004) |

### - Example according to the invention

The equipment shown on Figure 1 and described in relation thereto was used to reproduce the process according to this invention, wherein the reactor was a roundbottom vessel of 500 mL and the sample vials were 100 mL Erlenmeyer flasks.

### Example 1 (Comparative)

70 grams of diamide compound prepared in Synthesis example 1 and 280 grams of a mixture of solvents (xylene:ethanol, 3:1) were placed in a 500mL metal dish at room temperature (20°C). Using an ATP Dispermill disperser equipped with a paddle 3 cm in diameter, the products were mixed at a speed of 1500 revolutions/minute (or rpm) for 30 minutes at a temperature not exceeding 20°C, by regulating the temperature via circulation of cold water. The activation was carried out by carefully closing the dish and placing it in an oven preheated to 60°C for 24 hours. Once cooled and after 4 hours at rest, the paste was sufficiently hard to maintain a metal spatula in vertical position.

### Example 2 (According to the invention)

40 grams of diamide compound prepared in Synthesis Example 1 and 160 grams of a mixture of solvents (xylene:ethanol, 3:1) were placed in a round-bottomed vessel.

The flask was then placed at 5°C under mechanical stirring for 30 minutes.

The mixture was then pumped using a peristaltic pump to a coil distillate condenser preheated at 60°C.

At the other end of the tube, a paste was collected into sample vials, also preheated at 60°C. The sample vials were removed from the hot bath once they were filled. Optionally, in a semi-continuous process, the sample vials may then be placed in an oven preheated to 60°C for 1 hour. Once cooled and after 4 hours at rest, the paste was sufficiently hard to maintain a metal spatula in vertical position.

## Claims

1. A process for producing a pre-activated organogel, wherein the process comprises :
(1) mixing at least one amide compound with at least one liquid carrier so as to provide a mixture, wherein the mixing is carried out at a temperature T1 which is below the activation temperature of the amide compound,
(2) continuously flowing said mixture through a heat exchanger to increase its temperature to a temperature T2 that is at least equal to the activation temperature of said amide compound, so as to obtain an organogel,
(3) filling said organogel into containers maintained at or above said activation temperature, and
(4) retrieving and cooling said containers.

2. The process according to claim 1, **characterized in that** temperature T1 is at most 30°C, preferably at most 25°C, even more preferably from 0 to 25°C.

3. The process according to claim 1 or 2, **characterized in that** step (1) is carried out under stirring at 1000-3000 rpm, preferably 1500-2000 rpm.

4. The process according to claim 3, **characterized in that** stirring is carried out for 10 minutes to 1 h, for instance 15 to 30 minutes.

5. The process according to any one of claims 1 to 4, **characterized in that** temperature T2 is within the range of 35 to 120°C, for instance 50 to 100°C.

6. The process according to any one of claims 1 to 5, **characterized in that** the residence time of the mixture in the heat exchanger of step (2) is less than 1 hour, preferably from 30 seconds to 50 minutes, still preferably from 1 minute to 30 minutes.

7. The process according to any one of claims 1 to 6, **characterized in that** it further comprises a step of transferring the containers to an oven for completing the activation of the amide compound between steps (3) and (4).

8. The process according to any one of claims 1 to 7, **characterized in that** the amide compound is obtained by polycondensation between:
a) at least one amine selected from an aliphatic C₂ to C₂₄ monoamine and/or diamine, a cycloaliphatic C₆ to C₁₈ monoamine and/or diamine, an aromatic C₆ to C₁₈ monoamine and/or diamine, and combinations thereof,
b) at least one hydroxylated C₃ to C₃₆ monocarboxylic acid,
c) optionally, at least one saturated linear non-hydroxylated C₂ to C₁₈ monocarboxylic acid.

9. The process according to any one of claims 1 to 8, **characterized in that** said component (a) comprises at least one C₂ to C₂₄, in particular C₂ to C₁₂, more particularly C₂ to C₈ linear aliphatic diamine; and optionally at least one other diamine selected from a cycloaliphatic C₆ to C₁₈ diamine, an aromatic C₆ to C₁₈ diamine, and combinations thereof.

10. The process according to any one of claims 1 to 9, **characterized in that** component (b) comprises at least one C₁₆ to C₂₂ monohydroxylated monocarboxylic acid, preferably 14-hydroxyeicosanoic acid and/or 9-, 10- and/or 12-hydroxystearic acid (9-HSA, 10-HSA and/or 12-HSA).

11. The process according to any one of claims 1 to 10, **characterized in that** the amide compound is a diamide compound obtained by polycondensation between 12-hydroxystearic acid and 1,6-hexamethylenediamine.

12. The process according to any one of claims 1 to 11, **characterized in that** the liquid carrier comprises one or more plasticizers, one or more reactive solvents, one or more non-reactive solvents or mixtures thereof.

13. The process according to any one of claims 1 to 11, **characterized in that** the liquid carrier comprises a plasticizer, in particular a plasticizer selected from polar organic plasticizers bearing at least one ether, ester and/or epoxy group.

14. The process according to any one of claims 1 to 13, **characterized in that** the liquid carrier comprises a reactive solvent, in particular a reactive solvent selected from a (meth)acrylic monomer, a styrenic monomer, a vinylic monomer, an olefinic monomer, an unsaturated polyacid or a derivative thereof, and mixtures thereof.

15. The process according to any one of claims 1 to 14, **characterized in that** the liquid carrier comprises a non-reactive solvent, in particular a non-reactive solvent selected from: xylene; alcohols such as methanol, ethanol, butanol and benzyl alcohol ; cyclic saturated hydrocarbons such as cyclopentane, cyclohexane, methylcyclohexane, ethylcyclohexane, dimethylcyclohexane, trimethylcyclohexane and decalin ; alkylesters of monocarboxylic acids such as ethyl acetate, butyl acetate, hexyl acetate and heptyl acetate, ethyl propionate, amyl propionate and ethyl ethoxypropionate ; alkylesters of dicarboxylic acids, such as methyl glutarate, methyl succinate and methyl adipate ; and mixtures thereof.

## Patentansprüche

1. Verfahren zur Herstellung eines voraktivierten Organogels, wobei das Verfahren Folgendes umfasst:
(1) Mischen mindestens einer Amidverbindung mit mindestens einem flüssigen Träger zur Bereitstellung einer Mischung, wobei das Mischen bei einer Temperatur T1, die unterhalb der Aktivierungstemperatur der Amidverbindung liegt, durchgeführt wird,
(2) kontinuierliches Leiten der Mischung durch einen Wärmetauscher zur Erhöhung ihrer Temperatur auf eine Temperatur T2, die mindestens gleich der Aktivierungstemperatur der Amidverbindung ist, zum Erhalt eines Organogels,
(3) Füllen des Organogels in Behälter, die bei oder über der Aktivierungstemperatur gehalten werden, und
(4) Wiedergewinnen und Abkühlen der Behälter.

2. Verfahren nach Anspruch 1, **dadurch gekennzeichnet, dass** die Temperatur T1 höchstens 30° C, vorzugsweise höchstens 25 °C, noch weiter bevorzugt 0 bis 25 °C, beträgt.

3. Verfahren nach Anspruch 1 oder 2, **dadurch gekennzeichnet, dass** Schritt (1) unter Rühren bei 1000-3000 U/min, vorzugsweise 1500-2000 U/min, durchgeführt wird.

4. Verfahren nach Anspruch 3, **dadurch gekennzeichnet, dass** das Rühren 10 Minuten bis 1 h, beispielsweise 15 bis 30 Minuten, durchgeführt wird.

5. Verfahren nach einem der Ansprüche 1 bis 4, **dadurch gekennzeichnet, dass** die Temperatur T2 im Bereich von 35 bis 120 °C, beispielsweise 50 bis 100 °C, liegt.

6. Verfahren nach einem der Ansprüche 1 bis 5, **dadurch gekennzeichnet, dass** die Verweilzeit der Mischung in dem Wärmetauscher von Schritt (2) weniger als 1 Stunde, vorzugsweise 30 Sekunden bis 50 Minuten, weiter bevorzugt 1 Minute bis 30 Minuten, beträgt.

7. Verfahren nach einem der Ansprüche 1 bis 6, **dadurch gekennzeichnet, dass** es ferner einen Schritt des Überführens der Behälter in einen Ofen zum Vervollständigen der Aktivierung der Amidverbindung zwischen den Schritten (3) und (4) umfasst.

8. Verfahren nach einem der Ansprüche 1 bis 7, **dadurch gekennzeichnet, dass** die Amidverbindung erhalten wird durch Polykondensation zwischen:
a) mindestens einem Amin, das aus einem aliphatischen C₂-C₂₄-Monoamin und/oder -Diamin, einem cycloaliphatischen C₆-C₁₈-Monoamin und/oder -Diamin, einem aromatischen C₆-C₁₈-Monoamin und/oder -Diamin und Kombinationen davon ausgewählt ist,
b) mindestens einer hydroxylierten C₃- bis C₃₆-Monocarbonsäure,
c) gegebenenfalls mindestens einer gesättigten linearen, nicht hydroxylierten C₂- bis C₁₈-Monocarbonsäure.

9. Verfahren nach einem der Ansprüche 1 bis 8, **dadurch gekennzeichnet, dass** die Komponente (a) mindestens ein lineares aliphatisches C₂- bis C₂₄-, insbesondere C₂- bis C₁₂- und spezieller C₂- bis C₈-Diamin und gegebenenfalls mindestens ein anderes Diamin, das aus einem cycloaliphatischen C₆- bis C₁₈-Diamin, einem aromatischen C₆- bis C₁₈-Diamin und Kombinationen davon ausgewählt ist, umfasst.

10. Verfahren nach einem der Ansprüche 1 bis 9, **dadurch gekennzeichnet, dass** Komponente (b) mindestens eine monohydroxylierte C₁₆- bis C₂₂-Monocarbonsäure, vorzugsweise 14-Hydroxyeicosansäure und/oder 9-, 10- und/oder 12-Hydroxystearinsäure (9-HSA, 10-HSA und/oder 12-HSA), umfasst.

11. Verfahren nach einem der Ansprüche 1 bis 10, **dadurch gekennzeichnet, dass** es sich bei der Amidverbindung um eine durch Polykondensation zwischen 12-Hydroxystearinsäure und 1,6-Hexamethylendiamin erhaltene Diamidverbindung handelt.

12. Verfahren nach einem der Ansprüche 1 bis 11, **dadurch gekennzeichnet, dass** der flüssige Träger einen oder mehrere Weichmacher, ein oder mehrere reaktive Lösungsmittel, ein oder mehrere nicht reaktive Lösungsmittel oder Mischungen davon umfasst.

13. Verfahren nach einem der Ansprüche 1 bis 11, **dadurch gekennzeichnet, dass** der flüssige Träger einen Weichmacher umfasst, insbesondere einen Weichmacher, der aus polaren organischen Weichmachern mit mindestens einer Ether-, Ester- und/oder Epoxygruppe, ausgewählt ist.

14. Verfahren nach einem der Ansprüche 1 bis 13, **dadurch gekennzeichnet, dass** der flüssige Träger ein reaktives Lösungsmittel umfasst, insbesondere ein reaktives Lösungsmittel, das aus einem (Meth)acrylmonomer, einem Styrolmonomer, einem vinylischen Monomer, einem olefinischen Monomer, einer ungesättigten Polysäure oder einem Derivat davon und Mischungen davon ausgewählt ist.

15. Verfahren nach einem der Ansprüche 1 bis 14, **dadurch gekennzeichnet, dass** der flüssige Träger ein nicht reaktives Lösungsmittel umfasst, insbesondere ein nicht reaktives Lösungsmittel, das aus Xylol; Alkoholen wie Methanol, Ethanol, Butanol und Benzylalkohol; cyclischen gesättigten Kohlenwasserstoffen wie Cyclopentan, Cyclohexan, Methylcyclohexan, Ethylcyclohexan, Dimethylcyclohexan, Trimethylcyclohexan und Decalin; Alkylestern von Monocarbonsäuren wie Essigsäureethylester, Essisgsäurebutylester, Essigsäurehexylester und Essigsäureheptylester, Propionsäureethylester, Propionsäureamylester und Ethoxypropionsäureethylester; Alkylestern von Dicarbonsäuren wie Glutarsäuremethylester, Bernsteinsäuremethylester und Adipinsäuremethylester und Mischungen davon ausgewählt ist.

## Revendications

1. Procédé de production d'un organogel pré-activé, dans lequel le procédé comprend :
(1) le mélange d'au moins un composé amide avec au moins un support liquide de façon à fournir un mélange, où le mélange est effectué à une température T1 qui est inférieure à la température d'activation du composé amide,
(2) l'écoulement de manière continue dudit mélange dans un échangeur de chaleur pour augmenter sa température jusqu'à une température T2 qui est au moins égale à la température d'activation dudit composé amide, de manière à obtenir un organogel,
(3) le remplissage dudit organogel dans des récipients maintenus à ladite température d'activation ou au-dessus, et
(4) la récupération et le refroidissement desdits récipients.

2. Procédé selon la revendication 1, **caractérisé en ce que** la température T1 est d'au plus 30 °C, préférablement d'au plus 25 °C, encore plus préférablement de 0 à 25 °C.

3. Procédé selon la revendication 1 ou 2, **caractérisé en ce que** l'étape (1) est conduite sous agitation à 1 000-3 000 tpm, de préférence à 1 500-2 000 tpm.

4. Procédé selon la revendication 3, **caractérisé en ce que** l'agitation est réalisée pendant 10 minutes à 1 h, par exemple 15 à 30 minutes.

5. Procédé selon l'une quelconque des revendications 1 à 4, **caractérisé en ce que** la température T2 est dans la plage de 35 à 120 °C, par exemple 50 à 100 °C.

6. Procédé selon l'une quelconque des revendications 1 à 5, **caractérisé en ce que** le temps de séjour du mélange dans l'échangeur de chaleur de l'étape (2) est inférieur à 1 heure, de préférence de 30 secondes à 50 minutes, encore de préférence de 1 minute à 30 minutes.

7. Procédé selon l'une quelconque des revendications 1 à 6, **caractérisé en ce qu'**il comprend en outre une étape de transfert des récipients dans un four pour terminer l'activation du composé amide entre les étapes (3) et (4).

8. Procédé selon l'une quelconque des revendications 1 à 7, **caractérisé en ce que** le composé amide est obtenu par polycondensation entre :
a) au moins une amine sélectionnée parmi une monoamine et/ou diamine aliphatique en C₂ à C₂₄, une monoamine et/ou diamine cycloaliphatique en C₆ à C₁₈, une monoamine et/ou diamine aromatique en C₆ à C₁₈, et des combinaisons correspondantes ;
b) au moins un acide monocarboxylique hydroxylé en C₃ à C₃₆,
c) éventuellement, au moins un acide monocarboxylique en C₂ à C₁₈ non hydroxylé, saturé linéaire.

9. Procédé selon l'une quelconque des revendications 1 à 8, **caractérisé en ce que** ledit composant (a) comprend au moins une diamine aliphatique linéaire en C₂ à C₂₄, en particulier en C₂ à C₁₂, plus particulièrement en C₂ à C₈ ; et éventuellement au moins une autre diamine choisie parmi une diamine cycloaliphatique en C₆ à C₁₈, une diamine aromatique en C₆ à C₁₈ et des combinaisons correspondantes.

10. Procédé selon l'une quelconque des revendications 1 à 9, **caractérisé en ce que** le composant (b) comprend au moins un acide monocarboxylique monohydroxylé C₁₆ à C₂₂, de préférence l'acide 14-hydroxyeicosanoïque et/ou l'acide 9-, 10- et/ou 12-hydroxystéarique (9-HSA, 10-HSA et/ou 12-HSA).

11. Procédé selon l'une quelconque des revendications 1 à 10, **caractérisé en ce que** le composé amide est un composé diamide obtenu par polycondensation entre l'acide 12-hydroxystéarique et la 1,6-hexaméthylènediamine.

12. Procédé selon l'une quelconque des revendications 1 à 11, **caractérisé en ce que** le support liquide comprend un ou plusieurs plastifiants, un ou plusieurs solvants réactifs, un ou plusieurs solvants non réactifs ou des mélanges correspondants.

13. Procédé selon l'une quelconque des revendications 1 à 11, **caractérisé en ce que** le support liquide comprend un plastifiant, en particulier un plastifiant choisi parmi les plastifiants organiques polaires portant au moins un groupe éther, ester et/ou époxy.

14. Procédé selon l'une quelconque des revendications 1 à 13, **caractérisé en ce que** le support liquide comprend un solvant réactif, en particulier un solvant réactif choisi parmi un monomère (méth)acrylique, un monomère styrénique, un monomère vinylique, un monomère oléfinique, un polyacide insaturé ou un dérivé de celui-ci, et des mélanges correspondants.

15. Procédé selon l'une quelconque des revendications 1 à 14, **caractérisé en ce que** le support liquide comprend un solvant non réactif, en particulier un solvant non réactif choisi parmi : un xylène ; des alcools tels que le méthanol, l'éthanol, le butanol et l'alcool benzylique ; des hydrocarbures saturés cycliques tels que le cyclopentane, le cyclohexane, le méthylcyclohexane, l'éthylcyclohexane, le diméthylcyclohexane, le triméthylcyclohexane et la décaline ; des esters d'alkyle d'acides monocarboxyliques tels que l'acétate d'éthyle, l'acétate de butyle, l'acétate d'hexyle et l'acétate d'heptyle, le propionate d'éthyle, le propionate d'amyle et l'éthoxypropionate d'éthyle ; des esters d'alkyle d'acides dicarboxyliques, tels que le glutarate de méthyle, le succinate de méthyle et l'adipate de méthyle ; et des mélanges correspondants.
